# FASCICULE DE BREVET EUROPEEN

(11) **EP 2 222 260 B1**
(45) Date de publication et mention de la délivrance du brevet: **11.05.2011**
(21) Numéro de dépôt: 08864543.7
(22) Date de dépôt: 19.12.2008
(51) Int. Cl.: A61F 11/08

(54) **DISPOSITIF ACOUSTIQUE D'ATTENUATION LINEAIRE DU SON PERCU**
AKUSTISCHE VORRICHTUNG FÜR LINEARE SCHALLSCHWÄCHUNG VON WAHRGENOMMENEN GERÄUSCHEN
ACOUSTIC DEVICE FOR LINEAR PERCEIVED-SOUND ATTENUATION

(30) Priorité: 21.12.2007 FR 0760183; 14.04.2008 FR 0852492
(43) Date de publication de la demande: 01.09.2010
(73) Titulaire: Earsonics, 34830 Clapiers (FR)
(72) Inventeur: LOPEZ, Franck, F-34170 Castelnau Le Lez (FR); ANGOT, Priscille, F-30250 Sommieres (FR)
(74) Mandataire: Rhein, Alain
(86) Numéro de dépôt international: PCT/FR2008/052382
(87) Numéro de publication internationale: WO 2009/081067

(56) Documents cités:
- WO-A1-98/31193
- WO-A2-02/084982
- US-A- 4 852 683
- US-A- 5 113 967
- ANONYME: "Les protections auditives pour une pratique musicale" ARTICLE INTERNET, [Online] 22 février 2006 (2006-02-22), pages 1-4, XP002529700 Extrait de l'Internet: URL:http://pinceoreilles.free.fr/les_prote ctions.pdf> [extrait le 2009-05-29]

## Description

La présente invention entre dans le domaine auditif, en particulier dans le cadre de la protection auditive.

L'invention a pour objet un dispositif d'atténuation acoustique, de type filtre antibruit.

Un tel dispositif trouvera une application toute particulière en tant que prothèse auditive réalisée sur mesure mais aussi dans le cadre de bouchon antibruit.

Plus spécifiquement, la présente invention vise une atténuation linéaire du son perçu, à l'inverse des bouchons et filtres atténuant le son de manière sélective, uniquement au niveau d'une ou plusieurs parties du spectre auditif. L'invention se veut à même de restituer, atténué, l'intégralité du spectre acoustique, notamment les fréquences hautes correspondant aux médiums et aigus.

L'état de la technique connait déjà des filtres de prothèse auditive ayant une atténuation linéaire du son perçu. Toutefois, les filtres existants ne permettent pas une atténuation linéaire sur toute la plage de fréquences du spectre acoustique. En particulier, les fréquences audibles supérieures à 8000 Hz sont fortement atténuées après filtration, générant une chute brutale sur cet intervalle du spectre au-delà de cette borne. Dans le pire des cas, il peut s'agir d'un écrêtage engendrant une disparition total des fréquences au-delà de ladite borne.

De plus, un autre inconvénient des dispositifs de filtration existants réside dans le fait qu'ils ne tiennent aucunement compte des critères psycho-acoustique d'interprétation des sonorités. Là où l'acoustique étudie la nature et les propriétés des ondes sonores, la psycho-acoustique concerne la façon dont elles sont captées par le système auditif et la manière dont elles sont interprétées par le cerveau. De cette étude, on déduit que la perception des caractéristiques d'un son n'a pas de valeurs de mesure objectives.

En particulier, l'écrêtage des fréquences élevées détruit les harmoniques paires situées de 10 à 16 kHz, ces dernières contribuant à une sonorité agréable ou un son dit « musical ».

La présente invention, telle qu'elle est revendiquée, a pour but de pallier les inconvénients de l'état de la technique en proposant un dispositif acoustique d'atténuation linéaire du son sur une large bande du spectre. L'invention se veut à même de contrôler le niveau d'atténuation et conserver une réponse linéaire après filtration pour un meilleur confort d'écoute.

En particulier, l'invention offre la possibilité d'atténuer un son sans perte des harmoniques et des fréquences élevées. L'invention produit un son filtré atténué comparable à un effet « loudness », à savoir une correction en fréquence qui permet d'obtenir subjectivement un son plus puissant lors d'une écoute à bas volume.

Pour illustrer la différence entre les dispositifs existants et le dispositif selon l'invention, il est fait référence à la figure A. Cette dernière représente l'atténuation en décibel (DB) en fonction du spectre acoustique, exprimé en Hertz (Hz).

La courbe la plus basse, référencée G, correspond à l'atténuation d'un bouchon, par exemple en mousse. Les courbes D, E et F transcrivent l'atténuation non linéaire des dispositifs connus. On constate une baisse similaire à l'atténuation d'un bouchon. Même si la courbe D, qui correspond à un bouchon réalisé sur mesure par moulage, remonte à partir de 4000 Hz, on constate une perte totale des fréquences les plus aigues au-delà de 8000 Hz.

Un exemple d'un tel dispositif d'atténuation non linéaire est décrit dans le document EP 0 112 594, dans lequel une prothèse auditive moulée reçoit un dispositif de filtration non linéaire. Ce dernier comporte un canal véhiculant le son depuis une entrée vers une sortie, la première portion dudit canal étant amovible de manière à permettre l'insertion d'un filtre au niveau de l'extrémité de sa seconde portion.

En raison du faible diamètre du canal, une cavité peut être ménagée de manière à éviter l'obstruction par des particules de poussière ou de graisse. Toutefois, cette cavité peut engendrer des perturbations acoustiques non contrôlées.

De plus, la faible section dudit canal atténue considérablement le spectre acoustique en moyenne fréquence et écrête totalement les fréquences hautes, au-delà de 8000kHz.

Le document US 5113967 décrit un dispositif utilisant un système d'amplification, de filtrage et d'atténuation dans un ordre différent de celui de l'invention. Le résultat est un effet similaire, mais limité à la zone de fréquences 500 - 6 000 Hz

La courbe C correspond à un dispositif de filtration linéaire précédemment évoqué. On constate que la courbe est en moyenne croissante jusqu'à 4kHz puis décroissante.

Au contraire, les courbes A et B, représentant l'atténuation de deux modes de réalisations de dispositif de filtration selon l'invention, sont inversées, générant l'effet acoustique dit de « loudness », agréable à l'oreille puisqu'une telle courbe respecte l'équilibre tonal de l'oreille humaine.

Pour ce faire, la présente invention combine des technologies opposées consistant à, d'une part, atténuer linéairement le son perçu et, d'autre part, obtenir une résonance de ce dernier. Qui plus est, le son est amplifié en sortie du dispositif selon l'invention, au travers d'un phénomène de projection afin d'accentuer les fréquences à ondes courtes, soient les mediums et aigus. Ainsi, l'invention permet d'atténuer linéairement un son perçu, sans perdre la sonorité et la qualité de ce dernier, en restituant les fréquences hautes, notamment au-delà de 8kHz. On obtient donc un effet d'atténuation « loudness » plus naturel à l'oreille, au travers d'une atténuation plus forte dans les fréquences basses plus sensibles et en relevant les fréquences élevées, offrant une perception linéaire.

De plus, une réalisation particulière dans le cadre de prothèse moulée sur mesure permet d'améliorer considérablement la qualité audio, obtenant un son plus naturel et musical.

L'invention telle qu'elle est décrite dans la revendication 1 a donc pour objet un dispositif acoustique d'atténuation linéaire du son perçu, comprenant un canal véhiculant ledit son depuis une entrée jusqu'à une sortie, caractérisé par le fait qu'il comprend, combinés de manière à conférer une atténuation linéaire du son perçu, en entrée, des moyens d'atténuation et de mise en résonance dudit son véhiculant ce dernier jusqu'à au moins un filtre débouchant, en sortie, sur des moyens d'accentuation du son filtré, en particulier ses fréquences à ondes courtes, lesdits moyens d'accentuation étant constitués d'une portion dudit canal de forme tronconique de section croissante depuis ledit logement vers ladite sortie.

L'invention telle qu'elle est décrite dans la revendication 7 concerne aussi un bouchon antibruit, comprenant un tel dispositif acoustique.

L'invention telle qu'elle est décrite dans la revendication 8 concerne encore une prothèse auditive, réalisée par moulage sur mesure et conformée de manière à recevoir et recevant ledit dispositif acoustique

D'autres caractéristiques et avantages de l'invention ressortiront de la description détaillée qui va suivre des modes de réalisation non limitatifs de l'invention, en référence aux figures annexées dans lesquelles :
- la figure A est une représentation comparatives des courbes d'atténuation des dispositifs de l'état de la technique par rapport à l'invention.
- la figure 1 représente une vue en perspective d'un dispositif acoustique selon un mode de réalisation de l'invention ;
- la figure 2 représente une vue de coupe du dispositif de la figure 1 ;
- la figure 3 représente une vue en coupe du dispositif de la figure 1 selon un autre mode de réalisation.
- la figure 4 représente une vue en perspective d'un dispositif acoustique selon un autre mode de réalisation de l'invention ; et
- la figure 5 représente une vue de coupe du dispositif de la figure 4.

La présente invention concerne un dispositif acoustique d'atténuation linéaire du son perçu.

De façon préférentielle mais non limitative, selon les modes de réalisation visibles sur les dessins, un tel dispositif présente une forme générale cylindrique.

Il comprend un canal 1 ménagé à l'intérieur dudit dispositif, traversant ce dernier longitudinalement. Ce canal 1 véhicule le son depuis une entrée 2 jusqu'à une sortie 3 situées à chacune des extrémités dudit canal 1, opposées l'une à l'autre.

Tout au long de ce canal, l'invention prévoit des moyens aptes à atténuer, mettre en résonance, filtrer et accentuer ledit son, et dont la combinaison permet une restitution atténuée linéairement de l'intégralité du spectre acoustique, en particulier une atténuation des fréquences hautes, notamment au-delà de 8kHz.

En d'autres termes, le dispositif selon l'invention accorde le son, à savoir qu'il prend le son dans son contenu fréquentiel tel qu'il arrive en entrée 2, maintien ce contenu spectral tout en l'atténuant puis l'égalise. Le résultat, proche d'un effet « loudness » est obtenu en réduisant le son comme le ferait naturellement l'oreille, tout en compensant les fréquences extrêmes, à savoir en excitant les fréquences en-dessous de 2kHz et au-dessus de 8kHz.

Pour ce faire, en entrée 3, ledit dispositif comprend des moyens 4 d'atténuation et de mise en résonance du son. De manière préférentielle, ces moyens 4 sont constitués par une première portion dudit canal 1 ouverte sur l'extérieur au niveau de l'entrée 3. Les dimensions de cette portion modifient l'atténuation ou la mise en résonance du son perçu. Par conséquent, la longueur des moyens 4 est déterminée en fonction de la résonance souhaitée. De même, la section des moyens 4 est déterminée en fonction de l'atténuation souhaitée.

De préférence, mais non limitativement, les moyens 4 permettent d'accentuer les aigües avant filtration.

Ce résultat peut être obtenu au travers d'une largeur de section importante et constante jusqu'à la filtration. En particulier, cette largeur doit être rapportée à sa longueur pour maintenir un rapport permettant la mise en résonance et le maintien en résonance des ondes jusqu'à filtration.

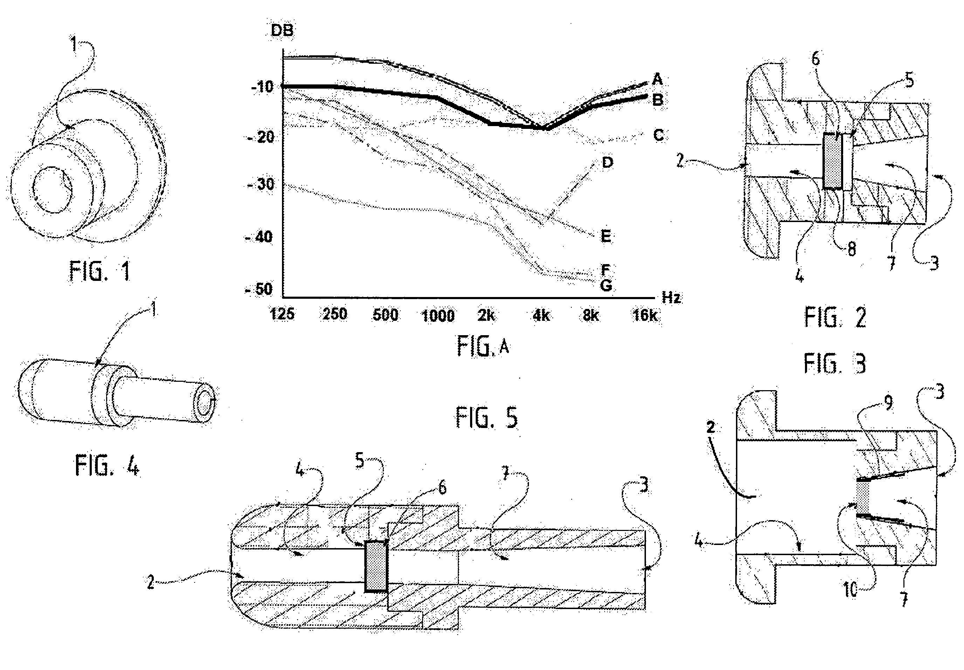

A titre d'exemple non limitatif, visible sur la figure 3, la première portion du canal 1 constituant les moyens 4 peut présenter une section intérieure circulaire d'un diamètre de 4 mm tandis qu'elle présente une longueur de 4,5 mm entre l'entrée 2 et la filtration. Pour obtenir un effet similaire de résonances des fréquences, ce mode de réalisation prévoit une largeur plus importante par rapport à sa longueur moindre.

Comparé à un autre mode de réalisation, visible sur la figure 5, cette section peut mesurer 1,4 mm tandis que la longueur vaut 6,1 mm. Comme évoqué précédemment, la section est moins grande en raison d'une longueur plus importante.

Quoi qu'il en soit, la section minimale de l'entrée 2 du canal 1 doit atteindre au moins 0,5 mm de manière à capter une onde avec un spectre acoustique le plus large possible, notamment sans écrêtage des fréquences élevées. Comparé à l'état de la technique, cette largeur d'entrée 2 importante diminue l'atténuation par rapport à une section plus petite, de l'ordre du dixième de millimètre, de 0,1 à 0,2 mm.

De manière générale, les moyens 4 véhiculent le son jusqu'à des moyens de filtration.

Selon un mode de réalisation, visible sur la figure 2, ces moyens 4 débouchent au sein d'un logement 5 de réception desdits moyens de filtration sous forme d'au moins un filtre 6. Ce logement 5 se présente sous la forme d'une cavité de dimensions adaptées, d'une part, à recevoir ledit filtre 6 et, d'autre part, à maintenir le son en résonance. En l'occurrence, la largeur dudit logement 5 est équivalente à celle du filtre 6 de manière à maintenir ce dernier au sein de la cavité. La résonance s'effectue ici encore en fonction de la longueur dudit logement 5, avant et/ou après le filtre 6, mais aussi le long de ce dernier.

A ce titre, ledit filtre 6 est choisi en fonction des caractéristiques acoustiques voulues. Plus particulièrement, selon un mode non limitatif de réalisation, le filtre 6 comprend des dimensions standards. Il peut être de tout type, plus particulièrement de type résistance acoustique à impédance. Un tel filtre permet d'obtenir une courbe régulée, à la manière d'un « acoustic damper ».

Ce filtre, peut être placé en avant de la cavité, directement en sortie des moyens 4, au centre de celle-ci ou en arrière. Le positionnement du filtre 6 étant étudié de manière à modifier le son selon le résultat envisagé.

En somme, le logement 5 enfermant le filtre 6 joue un rôle de résonateur filtrant. On notera que le logement 5 enfermant le filtre 6 peut s'aligner avec la première portion du canal 1, formant les moyens d'atténuation 4, offrant une continuité dans le maintien de la résonance des fréquences.

Selon un autre mode de réalisation, visible sur la figure 3, ledit filtre 6 est placé en fin des moyens 4, au niveau de la sortie de ces derniers, de manière ce que le son débouche directement sur ledit filtre 6.

On constate ici que la section du canal 1 a été élargie de manière optimale pour améliorer la mise en résonance du son.

De plus, en sortie 3 dudit dispositif, sont prévus des moyens 7 d'accentuation du son filtré. Ces derniers sont disposés en sortie du logement 5 et reçoivent en entrée le son filtré.

Ces moyens 7 créent un effet de pavillonnage générant un phénomène de projection. Ils accentuent plus particulièrement les fréquences à ondes courtes, telles les médiums et aigus. Ces moyens 7 permettent donc d'exciter de nouveau les fréquences précédemment atténuées, contribuant à l'effet « loudness » et à la musicalité du son en sortie 3.

Pour ce faire, lesdits moyens 7 d'accentuation sont constitués par une portion dudit canal 1 de forme tronconique de section croissante depuis ledit logement 5 vers ladite sortie 3. La section et l'angle de conicité desdits moyens d'accentuation 7 sont déterminés en fonction de l'accentuation souhaitée du son.

De plus, l'angle de conicité peut varier selon la longueur. Comme évoqué précédemment pour les moyens d'atténuation 4, une longueur plus importante du cône permet d'obtenir avec un angle plus faible (figure 5) un résultat comparable avec un angle plus importante pour une longueur moindre (figure 3).

Dans le cas visible sur la figure 3, le filtre 6 est inséré directement en entrée desdits moyens d'accentuation 7. A ce titre, dans ce cas de figure, ledit filtre 6 peut être privé de son enveloppe protectrice 8 de sorte que son maintien s'effectue le long des parois des moyens 7 d'accentuation par les bords 9 de sa membrane 10, par appui en force.

D'autre part, en fonction des modes de réalisation envisagés, figures 2 ou 3, le dispositif selon l'invention peut être réalisé en une seule ou deux parties. En effet, l'insertion du filtre 6 au sein du logement 5 nécessite que le dispositif soit constitué des deux éléments coopérant par emboîtement.

Tandis que la configuration de la figure 3 permet d'insérer directement ledit filtre 6, par emboîtement en force, maintenu alors par la contrainte des parois desdits moyens d'accentuation 7. Il n'est alors plus nécessaire d'avoir deux éléments coopérant entre eux.

La présente invention concerne aussi un bouchon antibruit, comprenant un dispositif acoustique comme précédemment évoqué. De manière particulière, le bouchon en entier peut être constitué par ledit dispositif. Selon un autre mode de réalisation, ledit dispositif peut être inséré, enfermé ou entouré par ledit bouchon.

Un tel bouchon de dimensions standards s'adapte à tout type d'oreille. De plus, il peut être constitué d'un matériau à mémoire de forme, de type mousse expansée ou analogue, de manière à être en partie contraint puis à reprendre sa forme d'origine, se bloquant dans l'oreille de l'utilisateur et atténuant les sons parasites non filtrés par le dispositif selon l'invention.

La présente invention concerne encore une prothèse auditive, réalisée par moulage sur mesure. Adaptée à la taille de l'oreille d'un utilisateur, une telle prothèse empêche tout son parasite du fait qu'elle épouse parfaitement les contours internes de ladite oreille.

Une telle prothèse est conformée de manière à recevoir un dispositif acoustique selon l'invention et reçoit un tel dispositif. Ce dernier peut être amovible ou directement enveloppé par la prothèse lors de l'étape de moulage.

Plus particulièrement, dans un example ne faisant pas parti de l'invention, une telle prothèse peut comprendre en sortie un tube de section et longueur déterminées en fonction du résultat audio à obtenir. Ce tube contribue à obtenir un son atténué plus naturelle de perception des sonorités au travers d'un phénomène de réflexion des fréquences médium et aigu le long dudit tube.

La présente invention consiste donc en un protecteur auditif à même d'offrir une atténuation linéaire du son filtré avec des qualités d'écoute supérieures et un son plus naturel, au travers d'une combinaison de l'atténuation puis de l'amplification du son filtré, mis en résonance tout au long du dispositif.

Le dimensionnement spécifique, notamment au travers d'une largeur plus importante diminuant l'effet d'atténuation, permet de couvrir une largeur de spectre acoustique plus importante, allant jusqu'aux fréquences élevées, au-delà de 8kHz et même jusque 16kHz.

L'invention permet donc de restituer ces hautes fréquences perçues et filtrées, conférant un effet « loudness » au son en sortie 3.

Bien entendu, l'invention n'est pas limitée aux exemples illustrés et décrits précédemment qui peuvent présenter des variantes et modifications sans pour autant sortir du cadre de l'invention telle qu'elle est revendiquée.

## Revendications

1. Dispositif acoustique d'atténuation linéaire du son perçu, comprenant un canal (1) véhiculant ledit son depuis une entrée (2) jusqu'à une sortie (3), **caractérisé par le fait qu'**il comprend, combinés de manière à conférer une atténuation linéaire du son perçu, en entrée (2), des moyens (4) d'atténuation et de mise en résonance dudit son véhiculant ce dernier jusqu'à au moins un filtre (6), lesdits moyens (4) s'étendant sur une largeur de section constante jusqu'au sein d'un logement (5) de réception dudit filtre (6), ce dernier débouchant, en sortie (3), sur des moyens (7) d'accentuation du son filtré, en particulier ses fréquences à ondes courtes, lesdits moyens d'accentuation étant constitués d'une portion dudit canal (1) de forme tronconique de section croissante depuis ledit logement (5) vers ladite sortie (3).

2. Dispositif acoustique selon la revendication 1, **caractérisé par le fait que** lesdits moyens d'atténuation (4) sont constitués par une portion dudit canal (1) sur une longueur déterminée en fonction de la résonance souhaitée.

3. Dispositif acoustique selon l'une quelconque des revendications 1 ou 2, **caractérisé par le fait que** lesdits moyens d'atténuation (4) sont constitués par une portion dudit canal (1) selon une section déterminée en fonction de l'atténuation souhaitée.

4. Dispositif acoustique selon l'une quelconque des revendications précédentes, **caractérisé par le fait que**
ledit logement (5) se présente sous la forme d'une cavité de dimensions adaptées, d'une part, à recevoir ledit filtre (6) et, d'autre part, à maintenir le son en résonance.

5. Dispositif acoustique selon l'une quelconque des revendications précédentes, **caractérisé par le fait que** la longueur, la section et l'angle de conicité desdits moyens d'accentuation (7) sont déterminés en fonction de l'accentuation souhaitée.

6. Dispositif acoustique selon l'une quelconque des revendications précédentes, **caractérisé par le fait que** la section d'entrée (2) présente un diamètre d'au moins 0,5 millimètres.

7. Bouchon antibruit, comprenant un dispositif acoustique selon l'une quelconque des revendications précédentes.

8. Prothèse auditive, réalisée par moulage sur mesure et conformée de manière à recevoir et recevant un dispositif acoustique selon l'une quelconque des revendications 1 à 6.

9. Prothèse auditive selon la revendication 8, comprenant en sortie un tube de section et longueur déterminées en fonction du résultat audio à obtenir.

## Claims

1. Acoustic device for linear attenuation of the perceived sound, comprising a channel (1) conveying said sound from an entry (2) to an exit (3), wherein it comprises, combined so as to confer a linear attenuation of the perceived sound, at the entry (2), means (4) for attenuating and setting into resonance of said sound conveying the latter to at least one filter (6), said means (4) extending over a constant cross-section width to within a housing (5) designed for receiving said filter (6), the latter coming out, at the exit (3), onto means (7) for accentuating the filtered sound, in particular its short wave frequencies, said accentuation means consisting of a frustoconical portion of said channel (1) having a cross-section that increases from said housing (5) toward said exit (3).

2. Acoustic device according to claim 1, wherein said attenuation means (4) are formed of a portion of said channel (1) over a length determined according to the desired resonance desired.

3. Acoustic device according to any of the claims 1 or 2, wherein said attenuation means (4) are formed of a portion of said channel (1) according to a cross-section determined according to the desired attenuation.

4. Acoustic device according to any of the preceding claims, wherein said housing (5) is in the form of a cavity having appropriate dimensions, so as to, on the one hand, receive said filter (6) and, on the other hand, maintain the sound in resonance.

5. Acoustic device according to any of the preceding claims, wherein the length, the cross-section and the conicity angle of said accentuation means (7) are determined according to the desired accentuation.

6. Acoustic device according to any of the preceding claims, wherein the entry cross-section (2) has a diameter of at least 0.5 millimeters.

7. Earplug, comprising an acoustic device according to any of the preceding claims.

8. Hearing aid, made by molding to measure and formed so as to receive and receiving an acoustic device according to any of the claims 1 through 6.

9. Hearing aid according to claim 8, comprising, at the exit, a tube the cross-section and length of which are determined according to the audio result to be achieved.

## Patentansprüche

1. Akustische Vorrichtung zur linearen Dämpfung des wahrgenommenen Schalles, umfassend einen Kanal (1), durch welchen der besagte Schall von einem Eingang (2) bis zu einem Ausgang (3) übertragen wird, **dadurch gekennzeichnet, dass** er am Eingang (2) Mittel (4) zur Dämpfung und zum Setzen in Resonanz des besagten, sich in diesem letzteren zumindest bis zu einem Filter (6) ausbreitenden Schalles umfasst, die derart kombiniert sind, um eine lineare Dämpfung des wahrgenommenen Schalles zu erlauben, wobei die besagten Mittel (4) sich auf eine Breite mit gleich bleibendem Querschnitt bis zu einer Aussparung (5) für die Aufnahme des besagten Filters (6) erstrecken, wobei dieser letztere am Ausgang (3) in den Mitteln (7) zur Verstärkung des filtrierten Schalles, insbesondere seiner Kurzwellenfrequenzen, mündet, wobei die besagten Verstärkungsmittel durch einen kegelstumpfförmigen Abschnitt des besagten Kanals (1) mit einem ab der besagten Aussparung (5) in Richtung zu dem besagten Ausgang (3) wachsenden Querschnitt gebildet seien.

2. Akustische Vorrichtung nach Anspruch 1, **dadurch gekennzeichnet, dass** die besagten Dämpfungsmittel (4) durch einen Teil des besagten Kanals (1) auf einer in Abhängigkeit von der gewünschten Resonanz bestimmten Länge gebildet sind.

3. Akustische Vorrichtung nach irgendeinem der Ansprüche 1 oder 2, **dadurch gekennzeichnet, dass** die besagten Dämpfungsmittel (4) durch einen Abschnitt des besagten Kanals (1) nach einem in Abhängigkeit von der gewünschten Dämpfung bestimmten Querschnitt gebildet sind.

4. Akustische Vorrichtung nach irgendeinem der vorgehenden Ansprüche, **dadurch gekennzeichnet, dass** die besagte Aussparung (5) als ein Hohlraum mit angepassten Abmessungen ausgestaltet ist, um, einerseits, den besagten Filter (6) aufzunehmen, und, andererseits, den Schall in Resonanz zu halten.

5. Akustische Vorrichtung nach irgendeinem der vorgehenden Ansprüche, **dadurch gekennzeichnet, dass** die Länge, der Querschnitt und der Konizitätswinkel der besagten Verstärkungsmittel (7) in Abhängigkeit von der gewünschten Verstärkung bestimmt sind.

6. Akustische Vorrichtung nach irgendeinem der vorgehenden Ansprüche, **dadurch gekennzeichnet, dass** der Querschnitt des Eingangs (2) einen Durchmesser von wenigstens 0,5 Millimetern aufweist.

7. Lärmschutzstöpsel, umfassend eine akustische Vorrichtung nach irgendeinem der vorgehenden Ansprüche.

8. Hörprothese, die durch Abformung nach Maß angefertigt und derart angepasst ist, um eine akustische Vorrichtung nach irgendeinem der Ansprüche von 1 bis 6 aufzunehmen, und die eine solche aufnimmt.

9. Hörprothese nach Anspruch 8, umfassend am Ausgang eine Röhre mit einem Querschnitt und mit einer Länge, die in Abhängigkeit von dem zu erzielenden Klangbild bestimmt sind.
